(19) Europäisches Patentamt
European Patent Office
Office européen des brevets

(11) Publication number : **0 336 466 B1**

# (12) EUROPEAN PATENT SPECIFICATION

(45) Date of publication of patent specification : **30.12.92 Bulletin 92/53**

(51) Int. Cl.$^5$ : **C07D 487/06,** A61K 31/55, // (C07D487/06, 243:00, 235:00)

(21) Application number : **89200575.2**

(22) Date of filing : **08.03.89**

## (54) Antiviral tetrahydroimidazo (1,4) benzodiazepin-2-ones.

(30) Priority : **18.03.88 GB 8806449**

(43) Date of publication of application : **11.10.89 Bulletin 89/41**

(45) Publication of the grant of the patent : **30.12.92 Bulletin 92/53**

(84) Designated Contracting States : **AT BE CH DE ES FR GB GR IT LI LU NL SE**

(56) References cited :
**EP-A- 0 107 569**
**EUR. J. MED. CHEM. - CHIMICA THERAPEUTICA vol. 13, n 1, 1978, pages 53-59; P. GENESTE et al. "Recherches en série de l'midazo-(4,5,1-jk)-benzodiazèpine-1,4 et de l'imidazo-(1,5,4-ef)-benzodiazèpine-1,5"**

(73) Proprietor : **JANSSEN PHARMACEUTICA N.V.**
**Turnhoutseweg 30**
**B-2340 Beerse (BE)**

(72) Inventor : **Raeymaekers, Alfons H.M.**
**Aanbeeldstraat 1**
**B-2340-Beerse (BE)**
Inventor : **Van Gelder, Josephus L.H.**
**Bareelstraat 30**
**B-2450-Kasterlee (BE)**
Inventor : **Kukla, Michael J.**
**1551 Oak Hollow Drive**
**Maple Glen Pennsylvania (US)**
Inventor : **Breslin, Henry J.**
**11974 Muhlenburg Drive**
**Lansdale Pennsylvania (US)**
Inventor : **Janssen, Paul A.J.**
**Antwerpsesteenweg 20**
**B-2350-Vosselaar (BE)**

EP 0 336 466 B1

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

In the Eur. J. Med. Chem. 1978, 13, 53-59, there are described three tetrahydroimidazo[4,5,1-jk][1,4]benzodiazepines. The compounds of the present invention differ therefrom by the fact that the imidazo-moiety is substituted with an oxo group and that the present compounds show antiviral activity.

The present invention is concerned with tetrahydroimidazo[1,4]benzodiazepin-2-ones having the formula

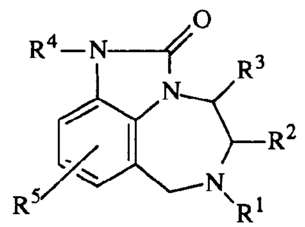

(I),

the pharmaceutically acceptable acid addition salts and the stereochemically isomeric forms thereof, wherein

$R^1$ is hydrogen, $C_{1-8}$alkyl, $C_{3-6}$alkenyl, $C_{3-6}$alkynyl, $C_{1-6}$alkylcarbonyl, $C_{3-6}$cycloalkyl, or $C_{1-6}$alkyl substituted with aryl, hydroxy, cyano or $C_{3-6}$cycloalkyl;

$R^2$ is hydrogen, $C_{1-6}$alkyl or $C_{3-6}$alkenyl;

$R^3$ is hydrogen or $C_{1-6}$alkyl;

$R^4$ is hydrogen; $C_{1-6}$alkyl optionally substituted with hydroxy, cyano, hydroxycarbonyl or $C_{1-6}$alkoxycarbonyl; $C_{1-6}$alkylcarbonyl; $C_{3-6}$alkenyl;

$C_{3-6}$cycloalkyl; $C_{5-6}$cycloalkenyl;

$R^5$ is hydrogen, $C_{1-6}$alkyl or halo; and

aryl is phenyl optionally substituted with up to 3 substituents independently selected from $C_{1-6}$alkyl, halo, hydroxy, $C_{1-6}$alkyloxy, amino, nitro and trifluoromethyl.

The compounds of formula (I) wherein $R^4$ is hydrogen, may exist in two tautomeric forms. Said tautomeric forms, although not explicitly indicated in the above formula, are intended to be included within the scope of the present invention.

In the foregoing definitions the term halo is generic to fluoro, chloro, bromo and iodo; $C_{1-6}$alkyl is meant to include straight and branched saturated hydrocarbon radicals having from 1 to 6 carbon atoms such as, for example, methyl, ethyl, propyl, 1-methylethyl, butyl, 1-methylpropyl, 2-methylpropyl, 1,1-dimethylethyl, pentyl, hexyl and the like; $C_{1-8}$alkyl defines $C_{1-6}$alkyl radicals and the higher homologs thereof containing 7 and 8 carbon atoms; $C_{3-6}$alkenyl defines straight and branched hydrocarbon radicals containing one double bond and having from 3 to 6 carbon atoms such as, for example, 2-propenyl, 2-butenyl, 3-butenyl, 2-methyl-2-propenyl, pentenyl, hexenyl and the like; $C_{3-6}$alkynyl defines straight and branch chained hydrocarbon radicals containing a triple bond and having from 3 to 6 carbon atoms such as, for example, 2-propynyl, 2-butynyl, 3-butynyl, pentynyl, hexynyl and the like; $C_{3-6}$cycloalkyl defines cyclopropyl, cyclobutyl, cyclopentyl and cyclohexyl; $C_{5-6}$cycloalkenyl defines cyclopentenyl and cyclohexenyl.

Depending on the nature of the various substituents the compounds of formula (I) may have several asymmetric carbon atoms. Unless otherwise mentioned or indicated, the chemical designation of compounds denotes the mixture of all possible stereochemically isomeric forms, said mixtures containing all diastereomers and enantiomers of the basic molecular structure. The absolute configuration of each chiral center may be indicated by the stereochemical descriptors R and S, this R and S notation corresponding to the rules described in Pure Appl. Chem. 1976, 45, 11-30. Stereochemically isomeric forms of the compounds of formula (I) are obviously intended to be embraced within the scope of the invention.

Pure stereochemically isomeric forms of the compounds of formula (I) may be obtained by the application of art-known procedures. Diastereoisomers may be separated by physical separation methods such as selective crystallization and chromatographic techniques, e.g., counter current distribution, liquid chromatography and the like; and enantiomers may be separated from each other by the selective crystallization of their diastereomeric salts with optically active acids. Pure stereochemically isomeric forms may also be derived from the corresponding pure stereochemically isomeric forms of the appropriate starting materials, provided that the reaction occurs stereospecifically.

The compounds of formula (I) have basic properties and, consequently, they may be converted to their therapeutically active non-toxic acid addition salt forms by treatment with appropriate acids, such as, for example, inorganic acids, e.g. hydrochloric, hydrobromic and the like acids, sulfuric acid, nitric acid, phosphoric acid and the like; or organic acids, such as, for example, acetic, propanoic, hydroxyacetic, 2-hydroxypropanoic, 2-oxopropanoic, ethanedioic, propanedioic, butanedioic, (Z)-2-butenedioic, (E)-2-butenedioic, 2-hydroxybutanedioic, 2,3-dihydroxybutanedioic, 2-hydroxy-1,2,3-propanetricarboxylic, methanesulfonic, ethanesulfonic, benzenesulfonic, 4-methylbenzenesulfonic, cyclohexanesulfamic, 2-hydroxybenzoic, 4-amino-2-hydroxybenzoic and the like acids. Conversely the salt form can be converted by treatment with alkali into the free base form. The term pharmaceutically acceptable acid addition salts also comprises the solvates which the compounds of formula (I) may form and said solvates are intended to be included within the scope of the present invention. Examples of such solvates are e.g. the hydrates, alcoholates and the like.

Particular compounds are those compounds of formula (I) wherein

$R^1$ is hydrogen, $C_{1-8}$alkyl, $C_{3-6}$alkenyl, $C_{3-6}$alkynyl, $C_{1-6}$alkylcarbonyl or $C_{1-6}$alkyl substituted with aryl, hydroxy, cyano or $C_{3-6}$cycloalkyl;

$R^2$ is hydrogen or $C_{1-6}$alkyl; and

$R^4$ is hydrogen; $C_{1-6}$alkyl optionally substituted with hydroxy; or $C_{1-6}$alkylcarbonyl.

More particular compounds are those particular compounds wherein

$R^1$ is $C_{1-8}$alkyl, $C_{3-6}$alkenyl, $C_{3-6}$alkynyl, or $C_{1-6}$alkyl substituted with aryl, hydroxy, cyano or $C_{3-6}$cycloalkyl;

$R^4$ is hydrogen; and

$R^5$ is hydrogen.

The most interesting compounds within the scope of the present invention are selected from 4,5,6,7-tetrahydro-5-methyl-6-(2-propenyl)imidazo[4,5,1-jk][1,4]benzodiazepin-2(1H)-one; 4,5,6,7-tetrahydro-5-methyl-6-(2-methyl-2-propenyl)imidazo[4,5,1-jk][1,4]benzodiazepin-2(1H)-one; (+)-(S)-4,5,6,7-tetrahydro-5-methyl-6-(2-propenyl)imidazo[4,5,1-jk]-[1,4]benzodiazepin-2(1H)-one; 6-(3-butenyl)-4,5,6,7-tetrahydro-5-methylimidazo-[4,5,1-jk][1,4]benzodiazepin-2(1H)-one; 4,5,6,7-tetrahydro-5-methyl-6-propylimidazo-[4,5,1-jk][1,4]benzodiazepin-2(1H)-one; (+)-(S)-4,5,6,7-tetrahydro-5-methyl-6-(2-methyl-2-propenyl)imidazo[4,5,1-jk][1,4]-benzodiazepin-2(1H)-one; 6-(cyclopropylmethyl)-4,5,6,7-tetrahydro-5-methylimidazo[4,5,1-jk][1,4]benzodiazepin-2(1H)-one monohydrate; and 4,5,6,7-tetrahydro-5-methyl-6-(3-methyl-2-butenyl)imidazo[4,5,1-jk][1,4]benzodiazepin-2(1H)-one.

The compounds of formula (I) can generally be prepared by condensing a 9-amino-2,3,4,5-tetrahydro-1H-1,4-benzodiazepine of formula (II) with a carbonyl-generating reagent of formula (III), wherein L is an appropriate leaving group.

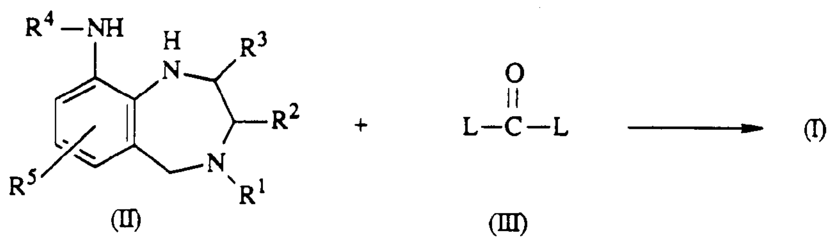

In formula (II) $R^1$, $R^2$, $R^3$, $R^4$ and $R^5$ are as defined in formula (I). Appropriate carbonyl-generating agents of formula (III) are for example urea, di$C_{1-6}$alkylcarbonate, carbonoic dichloride, trichloromethyl chloroformate, 1,1′-carbonylbis[1H-imidazole] and the like. Said condensation reaction may conveniently be conducted by stirring and optionally heating the reactants in a reaction-inert solvent, preferably having a relatively high boiling point, such as, for example, an aromatic hydrocarbon, e.g. benzene, methylbenzene, dimethylbenzene and the like; a halogenated hydrocarbon, e.g. trichloromethane, tetrachloromethane, chlorobenzene and the like; an ether, e.g. tetrahydrofuran, 1,4-dioxane, 1,1′-oxybisbutane, 1,1′-oxybis-(2-methoxyethane), 1,2-bis(2-methoxyethoxy)ethane and the like; a dipolar aprotic solvent, e.g. N,N-dimethylformamide, N,N-dimethylacetamide, dimethyl sulfoxide, 1-methyl-2-pyrrolidinone, pyridine, methylpyridine, dimethylpyridine, tetrahydrothiophene 1,1-dioxide and the like; or a mixture of such solvents. In some instances however, it may be preferable to heat the reactants without a solvent. Further it may be appropriate to add to the reaction mixture a base such as, for example, a tertiary amine, e.g. N,N-diethylethanamine, N-(1-methylethyl)-2-propanamine, 4-methylmorpholine and the like amines.

The compounds of formula (I) wherein $R^1$ is hydrogen, said compounds being represented by formula (I-a) can also be obtained from a benzylated compound of formula (I-b) following art-known hydrogenolysis procedures.

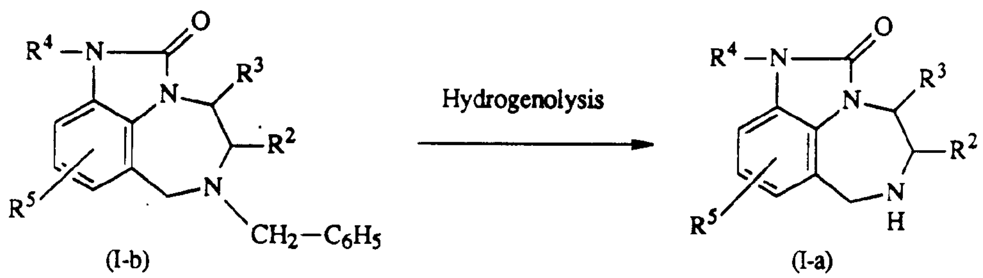


(I-b) (I-a)

In formulae (I-a) and (I-b), $R^2$, $R^3$, $R^4$ and $R^5$ have the previously defined meaning. Said debenzylation reaction can be accomplished by stirring a compound of formula (I-b) in an appropriate reaction-inert solvent in the presence of a suitable metal catalyst and under a hydrogen atmosphere. Appropriate solvents are, for example, alkanols, e.g. methanol, ethanol and the like; carboxylic esters, e.g. ethyl acetate; carboxylic acids, e.g. acetic acid, propanoic acid and the like. As examples of suitable metal catalysts there may be mentioned palladium-on-charcoal, platinum-on-charcoal and the like catalysts. In order to prevent the further hydrogenation of the starting material and/or the reaction product it may be appropriate to add a catalyst-poison to the reaction mixture such as, for example thiophene.

The compounds of formula (I) wherein $R^1$ is other than hydrogen, said $R^1$ being represented by $R^{1-a}$ and said compounds by formula (I-c) can also be obtained by N-alkylating a compound of formula (I-a) with a reagent of formula (IV). In formula (IV), W represents an appropriate reactive leaving group such as, for example, halo, e.g. chloro, bromo or iodo; or a sulfonyloxy group, e.g. benzenesulfonyloxy, 4-methylbenzenesulfonyloxy, methanesulfonyloxy and the like.

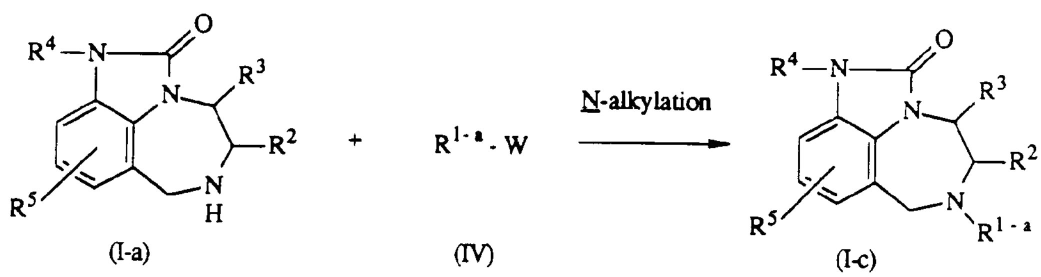


(I-a) (IV) (I-c)

Said N-alkylation reaction may conveniently be conducted in a reaction-inert solvent such as, for example, an aromatic hydrocarbon, e.g., benzene, methylbenzene, dimethylbenzene and the like; a lower alkanol, e.g., methanol, ethanol, 1-butanol and the like; a ketone, e.g., 2-propanone, 4-methyl-2-pentanone and the like; an ether, e.g., 1,4-dioxane, 1,1'-oxybisethane, tetrahydrofuran and the like; N,N-dimethylformamide; N,N-dimethylacetamide; nitrobenzene; dimethyl sulfoxide; 1-methyl-2-pyrrolidinone; and the like. The addition of an appropriate base such as, for example, an alkali metal carbonate or hydrogen carbonate, e.g. sodium carbonate, sodium hydrogen carbonate; sodium hydride or an organic base such as, for example, N,N-diethylethanamine or N-(1-methylethyl)-2-propanamine and the like may be utilized to pick up the acid which is liberated during the course of the reaction. In some circumstances the addition of an iodide salt, preferably an alkali metal iodide, e.g. potassium iodide, is appropriate. Somewhat elevated temperatures and stirring may enhance the rate of the reaction.

The compounds of formula (I-c) wherein $R^{1-a}$ is other than $C_{3-6}$alkenyl or $C_{3-6}$alkynyl and the carbon atom of said $R^{1-a}$ radical adjacent to the nitrogen atom bearing said $R^{1-a}$ contains at least one hydrogen atom, said radicals being represented by $R^{1-a-1}$, and said compounds by formula (I-d), may also be prepared by the reductive N-alkylation of a compound of formula (I-a) with a ketone or aldehyde of formula $R^{1-b}$=O (V). In formula (V), $R^{1-b}$ represents a geminal bivalent radical derived from $R^{1-a-1}$-H wherein two geminal hydrogen atoms are replaced by =O.

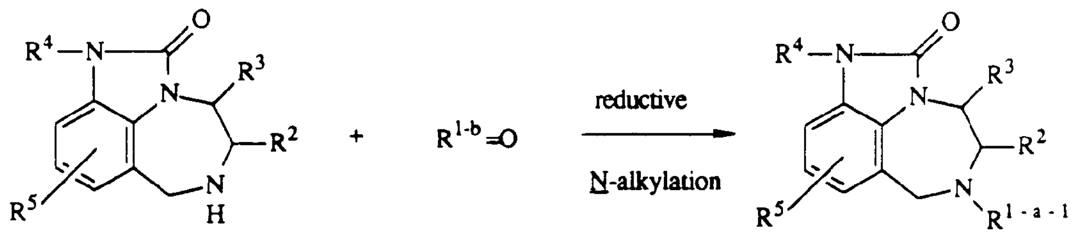


(I-a) (V) (I-d)

Said reductive N-alkylation reaction may conveniently be carried out by catalytically hydrogenating the reactants in a suitable reaction-inert organic solvent according to art-known catalytic hydrogenation procedures. The reaction mixture may be stirred and/or heated in order to enhance the reaction rate. Suitable solvents are, for example, water; $C_{1-6}$alkanols, e.g. methanol, ethanol, 2-propanol and the like; ethers, e.g. 1,4-dioxane and the like; halogenated hydrocarbons, e.g. trichloromethane and the like; N,N-dimethylformamide; dimethyl sulfoxide and the like; or a mixture of such solvents. The term "art-known catalytic hydrogenation procedures" means that the reaction is carried out under a hydrogen atmosphere and in the presence of an appropriate catalyst such as, for example, palladium-on-charcoal, platinum-on-charcoal and the like. In order to prevent the undesired further hydrogenation of certain functional groups in the reactants and the reaction products it may be advantageous to add an appropriate catalyst-poison to the reaction mixture, e.g., thiophene and the like. Alternatively, said reductive N-alkylation may also be performed following art-known reduction procedures by treating a stirred and, if desired, heated mixture of the reactants with a reducing agent such as, for example, sodium borohydride, sodium cyanoborohydride, formic acid or a salt thereof, in particular the ammonium salt thereof.

The compounds of formula (I) wherein $R^1$ is $R^{1-a}$ and $R^4$ is other than hydrogen, said $R^4$ being represented by $R^{4-a}$ and said compounds by formula (I-e), may be obtained by N-alkylating or N-acylating a compound of formula (I-f) wherein $R^4$ is hydrogen, with a reagent of formula (VI) wherein W is an appropriate reactive leaving group as defined hereinabove.

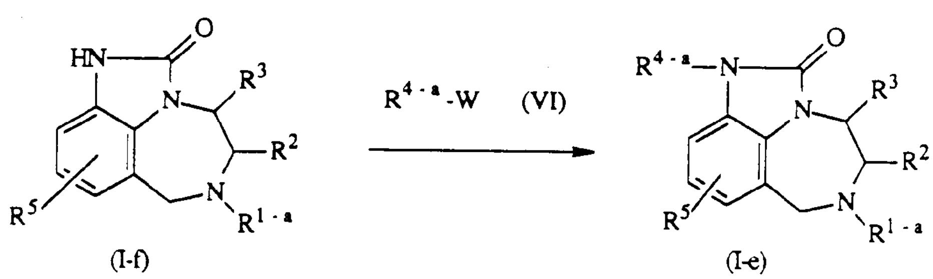


(I-f) (I-e)

Said N-alkylation and N-acylation reactions may be performed following art-known procedures for alkylating, respectively acylating amines.

In all of the foregoing and in the following preparations, the reaction products may be isolated from the reaction mixture and, if necessary, further purified according to methodologies generally known in the art.

A number of intermediates and starting materials in the foregoing preparations are known compounds which may be prepared according to art-known methodologies of preparing said or similar compounds and some intermediates are new. A number of such preparation methods will be described hereinafter in more detail.

The intermediates of formula (II) may generally be prepared from a 2,3,4,5-tetrahydro-9-nitro-1H-1,4-benzodiazepin-5-one of formula

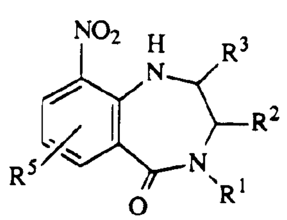

(VIII)

wherein $R^1$, $R^2$, $R^3$, and $R^5$ are as defined hereinabove, following art-known procedures for the reduction of nitro and amide groups to amine groups, e.g. by reacting the intermediates of formula (VIII) with a complex metal hydride such as lithium tetrahydroaluminate; a hydride such as, for example, diborane or aluminum hydride and the like, in a reaction-inert solvent such as, for example, 1,1′-oxybisethane, tetrahydrofuran, 1,4-dioxane, 1,2-dimethoxyethane and the like, optionally in the presence of co-solvent such as an aromatic hydrocarbon, e.g. benzene, methylbenzene and the like, and optionally at an elevated temperature.

The intermediates of formula (VIII) can be obtained from an appropriately substituted nitrobenzene (IX) by a condensation reaction with a diamino reagent of formula (X) in a suitable reaction-inert solvent such as, for example, an alkanol, e.g. methanol, ethanol, 2-propanol, 1-butanol and the like; an aromatic hydrocarbon, e.g. benzene, methylbenzene, dimethylbenzene and the like; a halogenated hydrocarbon, e.g. trichloromethane, tetrachloromethane and the like; an ether, e.g. tetrahydofuran, 1,4-dioxane, 1,1′-oxybisbutane, 1,1′-oxy(2-methoxyethane) and the like; a ketone, e.g. 2-propanone, 4-methyl-2-pentanone and the like; a dipolar aprotic solvent, e.g. N,N-dimethylformamide, N,N-dimethylacetamide, dimethyl sulfoxide and the like; or a mixture of such solvents. It may be appropriate to add a base such as an alkali metal or earth alkaline metal carbonate, e.g. sodium carbonate, sodium hydrogen carbonate and the like, to the reaction mixture.

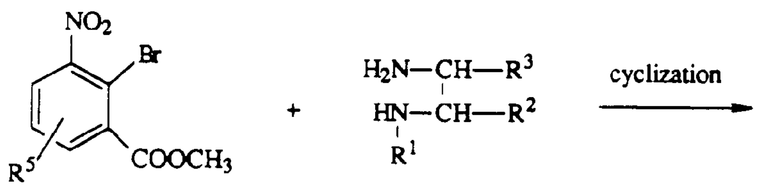

(VIII)

(IX) (X)

The intermediates of formula (II) wherein $R^1$, $R^3$ and $R^4$ are hydrogen, said intermediates being represented by formula

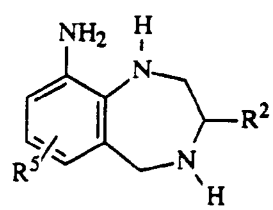
(II-a),

can be prepared from a benzodiazepindione of formula

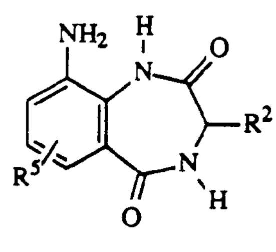
(XI),

following the reduction procedures as described hereinabove for converting intermediate (VIII) into intermediate (II).

The intermediates (XI) can be obtained by cyclizing an intermediate of formula

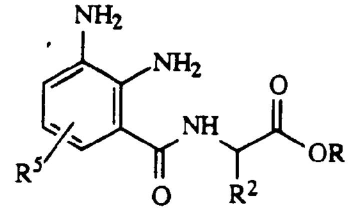

(XII),

wherein R represents a group such as $C_{1-6}$alkyl or aryl,

a) by heating (XII) without a solvent under an inert atmosphere, optionally under reduced pressure;

b) by treating (XII) with a bifunctional catalyst such as, for example, 2-hydroxypyridine, pyrazole, 1,2,4-triazole and the like, in a reaction-inert solvent such as, for example, an aromatic hydrocarbon, e.g. methylbenzene, dimethylbenzene and the like, optionally at an elevated temperature; or

c) by hydrolyzing the ester (XII) and subsequently treating the corresponding carboxylic acid (R = H) with an appropriate acid, such as, for example, a hydrohalic acid, e.g. hydrochloric acid; sulfuric acid, phosphoric acid and the like acids; or with a halogenating reagent such as, for example, thionyl chloride and the like.

The intermediates (XII) in turn can be prepared from the nitrobenzene

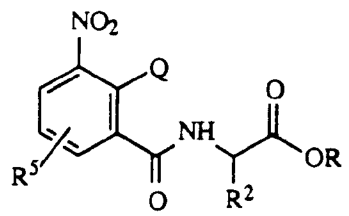

(XIII),

wherein Q may represent either amino or nitro, by catalytically reducing the nitro group(s) to amino group(s). Said catalytical reduction can conveniently be conducted by stirring the starting material in a reaction-inert solvent such as, for example, an alkanol, e.g. methanol, ethanol, propanol and the like, an ester, e.g. ethyl acetate, butyl acetate and the like, in the presence of hydrogen and an appropriate metal catalyst such as, for example, palladium-on-charcoal, Raney nickel and the like, optionally at an increased temperature and/or pressure. The intermediates (XIII) can be prepared from a suitably protected amino acid (XIV) and a benzoic acid (XV) wherein Q is either amino or nitro following art-known N-acylation procedures.

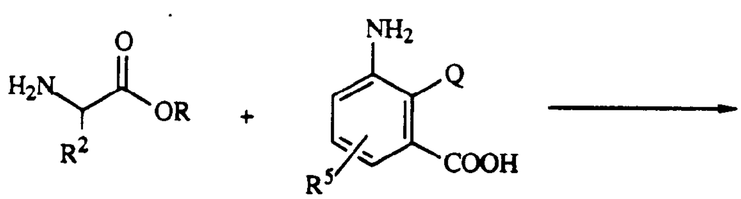

(XIV) + (XV) → (XIII)

Alternatively, the intermediates of formula (II-a) can be derived from the nitro derivative (XVI)

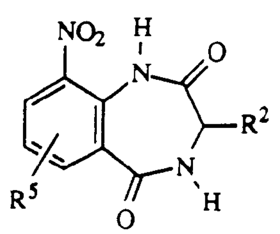

(XVI),

following the reduction procedures as described hereinabove for the preparation of (II) from (VIII). The intermediate (XVI) can be prepared by nitration of the benzodiazepindione (XVII) with concentrated nitric acid in the presence of concentrated sulfuric acid.

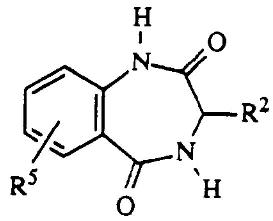

(XVII)

The intermediate (XVII) in turn can be prepared from an appropriately protected amino acid (XIV) and an intermediate (XVIII) by stirring the reactants at reflux temperature in an appropriate reaction-inert solvent such as, for example, trichloromethane, pyridine and the like.

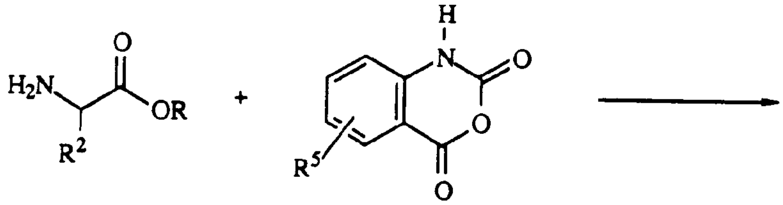

(XVII)

(XIV) (XVIII)

The intermediates of formula (II) wherein $R^1$ and $R^4$ are hydrogen and $R^3$ is $C_{1-6}$alkyl, said radical being represented by $R^{3-a}$ and said intermediates by formula

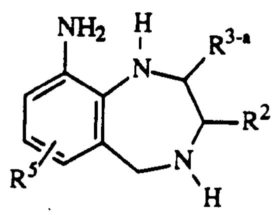

(II-b)

can be prepared by the reduction of an amine (XIX) or imine (XX), following the reduction procedures as described hereinabove for the preparation of (II) from (VIII).

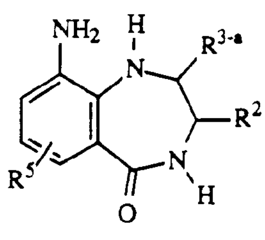

(XIX)

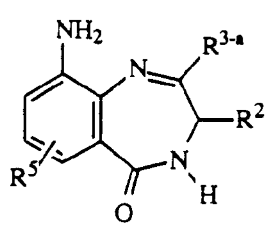

(XX)

The amine (XIX) and/or imine (XX) can be prepared by reducing a nitro derivative (XXI) in the presence of hydrogen and a suitable metal catalyst such as, for example, palladium-on-charcoal, platinum oxide and the like catalysts. The ketone of formula (XXI) in turn can be prepared from a 2-amino-3-nitrobenzoic acid (XXII) and an $\alpha$-aminoketone (XXIII) following art-known N-acylation procedures.

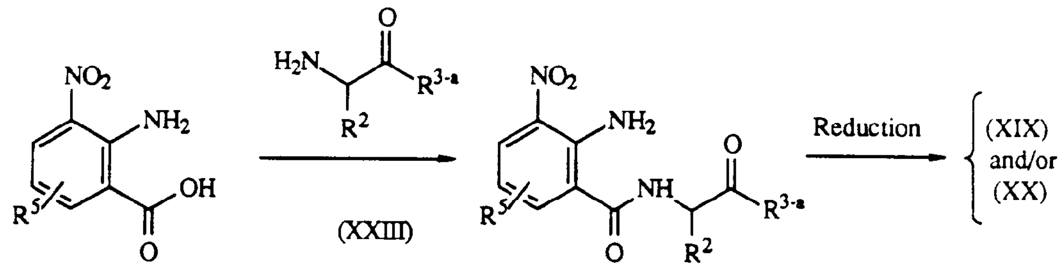


(XXIII)

(XIX) and/or (XX)

(XXII) (XXI)

The compounds of formula (I) show antiviral and in particular antiretroviral properties. Until recently, retroviruses were considered to be the pathogenic agents in a number of non-human warm-blooded animal diseases only, unlike viruses which have been known for quite some time to be the cause of a large number of diseases in warm-blooded animals and humans alike. However, since it has been established that a retrovirus, Human Immunodeficiency Virus (HIV), also known as LAV, HTLV-III or ARV, is the etiological agent of Acquired Immune Deficiency Syndrome (AIDS) in humans, retroviral infections and the treatment of subjects suffering therefrom have received the utmost attention. The HIV virus preferentially infects human T-4 cells and destroys them or changes their normal function, particularly the coordination of the immune system. As a result, an infected patient has an everdecreasing number of T-4 cells, which moreover behave abnormally. Hence, the immunological defense system is unable to combat infections and neoplasms and the HIV infected subject usually dies by opportunistic infections such as pneumonia, or by cancers, rather than as a direct result of HIV infections. Other conditions associated with HIV infection include thrombocytopaenia, Kaposi's sarcoma and infection of the central nervous system characterized by progressive demyelination, resulting in dementia and symptoms such as, progressive dysarthria, ataxia and disorientation. HIV infection further has also been associated with peripheral neuropathy, progressive generalized lymphadenopathy (PGL) and AIDS-related complex (ARC). The antiviral, in particular antiretroviral and especially the anti-HIV properties of the compounds of formula (I) suggest said compounds to be useful antiviral chemotherapeutical agents for the prophylaxis or treatment of warm-blooded animals suffering from viral infections.

The antiretroviral activity and cytotoxicity of the compounds of formula (I) can be determined using an in vitro anti-HIV assay system as described in the Journal of Virological Methods, 1987, 171-185. The 50% antiviral effective dose ($ED_{50}$; µg/ml) was obtained using the cytopathic effect (CPE) assay based on cell viability determined by the trypan blue dye exclusion method. The 50% cytotoxic dose ($CD_{50}$; µg/ml) was assayed in mock-infected MT-4 cells. For the compounds 17,22,23,25,27 and 33 (table on pages 20-21), the $CD_{50}$ was found to range from about 165 to about 250 µg/ml and the $ED_{50}$ from about 8 to about 22 µg/ml. No tranquilizing or sedative activity was observed.

Due to their antiviral and in particular their antiretroviral properties, the compounds of formula (I), their pharmaceutically acceptable salts and the stereochemically isomeric forms thereof, are useful in the treatment of warm-blooded animals infected with viruses, in particular retroviruses or for the prophylaxis of said warm-blooded animals. Examples of human retroviral infections include HIV and HTLV-I (human T-lymphotropic virus type I), causing leukemia and lymphoma. As an example of non-human animal retroviral infection there may be mentioned FeLV (feline leukemia virus) which causes leukemia and immunodeficiency. Conditions which may be prevented or treated with the compounds of the present invention, especially conditions associated with HIV and other pathogenic retroviruses, include AIDS, AIDS-related complex (ARC), progressive generalized lymphadenopathy (PGL), as well as chronic CNS diseases caused by retroviruses, such as, for example HIV mediated dementia and multiple sclerosis.

In view of their antiviral in particular antiretroviral activity, the subject compounds may be formulated into various pharmaceutical forms for administration purposes. To prepare the pharmaceutical compositions of this invention, an effective amount of the particular compound, in base or acid addition salt form, as the active ingredient is combined in intimate admixture with a pharmaceutically acceptable carrier, which carrier may take a wide variety of forms depending on the form of preparation desired for administration. These pharmaceutical compositions are desirably in unitary dosage form suitable, preferably, for administration orally, rectally, percutaneously, or by parenteral injection. For example, in preparing the compositions in oral dosage form, any of the usual pharmaceutical media may be employed, such as, for example, water, glycols, oils, alcohols and the like in the case of oral liquid preparations such as suspensions, syrups, elixirs and solutions: or solid carriers such as starches, sugars, kaolin, lubricants, binders, disintegrating agents and the like in the case of powders, pills, capsules and tablets. Because of their ease in administration, tablets and capsules represent the most

advantageous oral dosage unit form, in which case solid pharmaceutical carriers are obviously employed. For parenteral compositions, the carrier will usually comprise sterile water, at least in large part, though other ingredients, for example, to aid solubility, may be included. Injectable solutions, for example, may be prepared in which the carrier comprises saline solution, glucose solution or a mixture of saline and glucose solution. Injectable suspensions may also be prepared in which case appropriate liquid carriers, suspending agents and the like may be employed. In the compositions suitable for percutaneous administration, the carrier optionally comprises a penetration enhancing agent and/or a suitable setting agent, optionally combined with suitable additives of any nature in minor proportions, which additives do not cause a significant deleterious effect to the skin. Said additives may facilitate the administration to the skin and/or may be helpful for preparing the desired compositions. These compositions may be administered in various ways, e.g., as a transdermal patch, as a spot-on, as an ointment. Acid addition salts of (I) due to their increased water solubility over the corresponding base form, are obviously more suitable in the preparation of aqueous compositions.It is especially advantageous to formulate the aforementioned pharmaceutical compositions in dosage unit form for ease of administration and uniformity of dosage. Dosage unit form as used in the specification and claims herein refers to physically discrete units suitable as unitary dosages, each unit containing a predetermined quantity of active ingredient calculated to produce the desired therapeutic effect in association with the required pharmaceutical carrier. Examples of such dosage unit forms are tablets (including scored or coated tablets), capsules, pills, powder packets, wafers, injectable solutions or suspensions, teaspoonfuls, tablespoon-fuls and the like, and segregated multiples thereof.

The present invention is also related with a method of treating viral diseases in warm-blooded animals suffering from said viral diseases by administering an effective antiviral amount of compound of formula (I), a pharmaceutically acceptable acid addition salt or a stereoisomeric form thereof. Those of skill in the treatment of viral diseases could easily determine the effective antiviral amount from the test results presented herein. In general it is contemplated that an effective amount would be from 0.1 mg/kg to 200 mg/kg body weight, and in particular from 1 mg/kg to 50 mg/kg body weight. It may be appropriate to administer the required dose as two, three, four or more sub-doses at appropriate intervals throughout the day. Said sub-doses may be formulated as unit dosage forms, for example, containing 1 to 1000 mg, and in particular 5 to 200 mg of active ingredient per unit dosage form.

The following examples are intended to illustrate and not to limit the scope of the present invention in all its aspects. Unless otherwise stated all parts therein are by weight.

## Experimental part

### A) Preparation of intermediates

#### Example 1

a) A solution of 2.6 parts of methyl 2-bromo-3-nitrobenzoate, 1.75 parts of N-[(2-amino-1-methyl)ethyl]benzenemethanamine and 1.06 parts of sodium carbonate in 8 parts of 1-butanol was stirred and refluxed for 30 minutes. The solvent was evaporated. To the residue were added 20 parts of water and the product was extracted twice with 30 parts of trichloromethane. The combined extracts were dried, filtered and evaporated. From the oily free base, the hydrochloride salt was prepared in the usual manner. The salt was filtered off, washed with 2-propanol and dried, yielding 3.4 parts (89.5%) of methyl 3-nitro-2-[[2-methyl-2-[(phenylmethyl)amino]ethyl]amino]benzoate hydrochloride; mp. 204°C (intermediate 1).

b) A mixture of 3.8 parts of intermediate 1, 15 parts of a sodium hydroxide solution 2 N and 4 parts of 2-propanol was stirred and refluxed for one hour. To the boiling reaction mixture there was then added a solution of 3 parts of concentrated hydrochloric acid and 5 parts of water. After cooling, the product was precipitated. It was filtered off, washed with water and recrystallized from 80 parts of glacial acetic acid, yielding 3 parts (82%) of 3-nitro-2-[[[2-[(phenylmethyl)amino]-2-methyl]ethyl]amino]benzoic acid; mp. 227°C (intermediate 2).

c) A mixture of 189.3 parts of intermediate 2,400 parts of thionyl chloride and 400 parts of methylbenzene was stirred and refluxed for 2 hours. The solvent was evaporated and the residue was taken up in 600 parts of methylbenzene. The whole was treated with a sodium hydrogen carbonate solution. The separated organic layer was dried on anhydrous sodium carbonate, filtered and concentrated to a volume of about 500 parts. After standing at room temperature, the product partly precipitated. It was filtered off (the filtrate was set aside), washed successively with 2-propanol and 1,1'-oxybisethane and dried, yielding a first fraction of 123.5 parts of crude 2,3,4,5-tetrahydro-3-methyl-9-nitro-4-(phenylmethyl)-1H-1,4-benzodiazepin-5-one. From the mother liquor, the solvent was evaporated. The residue was dissolved in 160 parts of boiling 2-propanol and crystallized at room temperature. The precipitated product was filtered off, washed successively with 2-propanol and 1,1'-oxybisethane and dried, yielding a second less pure fraction of 28 parts of 2,3,4,5-tetrahydro-3-methyl-9-nitro-4-(phenyl-methyl)-1H-1,4-benzodiazepin-5-one. Both crude fractions were recrystallized from ethanol, yielding 137 parts (85%) of 2,3,4,5-tetrahydro-3-methyl-9-nitro-4-(phenylmethyl)-1H-1,4-benzodiazepin-5-one; mp. 125°C (intermediate 3).

d) To a stirred and refluxing suspension of 14 parts of lithium aluminium hydride in 40 parts of benzene and 50 parts of tetrahydrofuran was added a solution of 20.2 parts of intermediate 3 in 200 parts of tetrahydrofuran and the whole was further stirred and refluxed for 2.5 hours. The reaction mixture was cooled in crushed ice and decomposed by successive additions of water, sodium hydroxide solution 15% and again with water. The inorganic material was filtered off and the filtrate was evaporated. To the residue were added 40 parts of methylbenzene and this solution was evaporated to dryness, yielding 19.8 parts (87.6%) of 9-amino-2,3,4,5-tetrahydro-3-methyl-4-(phenylmethyl)-1H-1,4-benzodiazepin-5-one as a red-coloured, oily residue which was used without further purification for the preparation of the next step (intermediate 4).

Example 2

a) To a stirred and cooled (-12°C) mixture of 9.10 parts of 2-amino-3-nitrobenzoic acid, 6.95 parts of methyl (L)-2-aminopropanoate hydrochloride, 13.50 parts of 1-hydroxy-1H-benzotriazole hydrate and 180 parts of tetrahydrofuran were added 5.05 parts of 4-methyl-morpholine under an argon atmosphere. After stirring for 5 minutes, 10.30 parts of N,N-methanetetraylbis[cyclohexanamine] were added to the mixture. After 5.5 hours, the mixture was allowed to reach room temperature and stirred for 16 hours. The mixture was cooled for 30 minutes at 0°C and then filtered. The filtrate was concentrated under reduced pressure and the residue was partitioned between 225 parts of ethyl acetate and 250 parts of a saturated sodium hydrogen carbonate solution. The separated organic layer was washed with 100 parts of a saturated sodium hydrogen carbonate solution, dried, filtered and concentrated in vacuo, yielding 13.08 parts (97.9%) of (-)-methyl (S)-2-[(2-amino-3-nitrobenzoyl)amino]-propanoate; mp. 132.9°C (intermediate 5).

b) A mixture of 12.58 parts of intermediate 5 and 160 parts of ethanol was hydrogenated in a Parr apparatus at $3.10^5$ Pa and at room temperature with 3.50 parts of palladium-on-charcoal catalyst 10% during 4 hours. The catalyst was filtered off over diatomaceous earth and the filtrate was concentrated under reduced pressure. The oily residue was placed into an oil bath at 150°C at $3.3\ 10^3$ Pa. The temperature was remained at 200°C for 40 minutes while stirring. After cooling, the precipitated product was filtered off and triturated with 12 parts of ethanol. The product was filtered off, washed with a small amount of cold ethanol and 1,1'-oxybisethane and dried, yielding 5.58 parts (57.7%) of (+)-(S)-9-amino-2,3-dihydro-3-methyl-1H-1,4-benzodiazepine-2,5-(4H)-dione (intermediate 6).

Example 3

a) To a stirred solution of 11.32 parts of N,N-methanetetraylbis[cyclohexanamine] in 45 parts of tetrahydrofuran was added first a solution of 10 parts of 2-amino-3-nitrobenzoic acid and 7.42 parts of 1-hydroxy-1H-benzotriazole hydrate in 180 parts of tetrahydrofuran and then a solution of 5.55 parts of 4-methylmorpholine in 45 parts of tetrahydrofuran. A solution of 6 parts of 1-amino-2-propanone hydrochloride in 47 parts of N,N-dimethylformamide was added to the thus obtained mixture and the whole was stirred for 22 hours under a nitrogen atmosphere. An additional 1.5 parts of 1-amino-2-propanone hydrochloride and 1.4 parts of 4-methylmorpholine were added. The same amount of these products was added after stirring for 24 hours. After a total reaction time of 3 days, the whole was filtered and the filtrate was concentrated. The residue was dissolved in dichloromethane and the organic layer was washed successively twice with water, a saturated sodium hydrogen carbonate solution and a sodium chloride solution, dried, filtered and evaporated. The residue was crystallized from ethanol, yielding 8.34 parts (63.9%) of 2-amino-3-nitro-N-(2-oxopropyl)-benzamide (intermediate 7).

b) A mixture of 7.2 parts of intermediate 7 and 120 parts of ethanol was hydrogenated in a Parr apparatus at $3.5\ 10^5$ Pa with 1.7 parts of palladium-on-charcoal catalyst 10%. After the calculated amount of hydrogen was taken up, the catalyst was filtered off over diatomaceous earth, washed with dichloromethane and the filtrate was concentrated, yielding 5.5 parts (96.9%) of 9-amino-3,4-dihydro-2-methyl-5-H-1,4-benzodiazepin-5-one (intermediate 8).

B) Preparation of final compounds

Example 4

A mixture of 19.8 parts of intermediate 4 and 7.2 parts of urea was heated to a temperature between 210-220°C until foaming and evolution of gaseous ammonia ceased (about 10 minutes). The reaction was cooled to about 100°C and boiled with 120 parts of hydrochloric acid solution 1 N. The solution was decanted from the oily residue, treated with activated charcoal and filtered. The filtrate was cooled, alkalized with ammonium hydroxide and the product was extracted once with 75 parts of trichloromethane and once with 150 parts of trichloromethane. The combined extracts were dried and evaporated. The residue was triturated in 24 parts of 2-propanol, filtered off and recrystallized from ethanol and then from 4-methyl-2-pentanone, yielding 2.5 parts (11.5%) of 4,5,6,7-tetrahydro-5-methyl-6-(phenylmethyl)imidazo-[4,5,1-jk][1,4]benzodiazepin-2(1H)-one; mp. 205°C (compound 14).

Example 5

A mixture of 8 parts of compound 14, 1 part of palladium-on-charcoal catalyst 10% in 80 parts of glacial acetic acid was hydrogenated at about 38°C. After the calculated amount of hydrogen was taken up, the catalyst was filtered off and the acetic acid was evaporated. The residue was dissolved in 75 parts of water and the solution was alkalized with 30 parts of concentrated ammonium hydroxide solution. The product crystallized at room temperature. It was filtered off, washed with water and recrystallized from 20 parts of 2-propanol, yielding 3.7 parts (66.8%) of 4,5,6,7-tetrahydro-5-methyl-imidazo[4,5,1-jk][1,4]benzodiazepin-2(1H)-one; mp. 190.5°C (compound 11).

Example 6

At 25°C and under an argon stream, 5.0 parts of intermediate 6 were added to a suspension of 5.55 parts of lithium aluminum hydride in 154.5 parts of 1,4-dioxane. The reaction mixture was refluxed for 5 hours. After cooling to 10°C, 5.55 parts of water, 9.16 parts of a sodium hydroxide solution 15% and 16.65 parts of water were added successively. The whole was stirred for 2 hours and then filtered. The precipitate was washed successively with 178 parts of hot tetrahydrofuran and 133 parts of dichloromethane. The combined filtrates were dried, filtered and evaporated. The residue was poured into a solution of 7.36 parts of 4-methylmorpholine in 133 parts of dichloromethane. The whole was added to a solution of 4.82 parts of trichloromethyl chloroformate in 160 parts of dichloromethane over a period of 15 minutes at 0°C and under an argon stream. After stirring for 10 minutes at 0°C, the reaction mixture was warmed to room temperature and concentrated by evaporation. 70 Parts of an aqueous 1,4-dioxane solution (15%) were added to the residue and the mixture was heated on a steam-bath under a nitrogen stream for 45 minutes, cooled and extracted with dichloromethane (2 x 66.5 parts). The aqueous layer was filtered and basified with concentrated ammonium hydroxide. The precipitate was filtered off, washed with a small amount of cold water, dried and triturated twice with 6.24 parts of 2-propanol, yielding 1.59 parts (32.1%) of (+)-(S)-4,5,6,7-tetrahydro-5-methylimidazo[4,5,1-jk] [1,4]benzodiazepin-2(1H)-one; mp. 206.5°C (compound 12).

Example 7

A solution of 6.5 parts of compound 11, 4.65 parts of 3-bromo-1-propene, 3.85 parts of N,N-diethylethanamine and a few crystals of potassium iodide in 80 parts of 1-butanol was stirred and refluxed for 15 hours. The solvent was evaporated. To the residue were added 100 parts of water and the product was extracted twice with trichloromethane. The combined extracts were dried and evaporated. The residue was triturated in 160 parts of hot 2,2′-oxybispropane, boiled with activated charcoal, filtered and the filtrate was concentrated. The product was purified via the oxalate salt in the usual manner and the free base was liberated. The latter was first crystallized from 20 parts of 2,2′-oxybispropane and then from 12 parts of 2-propanol, yielding 2.5 parts (32.1%) of 4,5,6,7-tetrahydro-5-methyl-6-(2-propenyl)-imidazo[4,5,1-jk][1,4]benzodiazepin-2(1H)-one; mp. 138°C (compound 22).

Example 8

To a stirred solution of 1.0 part of compound 11, 0.816 parts of potassium iodide and 0.782 parts of sodium carbonate in 56.4 parts of N,N-dimethylformamide was added dropwise a solution of 0.88 parts of 4-bromo-2-methyl-1-butene in 14 parts of N,N-dimethylformamide. After stirring for 22.5 hours at room temperature,

the reaction mixture was concentrated in vacuo at ~70°C. The residue was partitioned twice between 130 parts of dichloromethane and 100 parts of a mixture of water and a saturated aqueous sodium hydrogen carbonate solution (50:50 by volume). The combined aqueous layers were extracted with 78 parts of dichloromethane. The dichloromethane layers were combined and extracted with 100 parts of a saturated sodium chloride solution. The extract was dried, filtered and concentrated in vacuo at ~40°C. The residue was crystallized twice from 16 parts of acetonitrile. The whole was cooled for 45 minutes at 0-5°C; the crystallized product was filtered off, washed with 4 parts cold (0-5°C) acetonitrile and dried overnight in vacuo at 78°C, yielding 0.805 parts (60.3%) of (±)-4,5,6,7-tetrahydro-5-methyl-6-(3-methyl-2-butenyl)imidazo[4,5,1-jk][1,4]benzodiazepin-2(1H)-one; mp. 158.0°C (compound 28).

Example 9

To a stirred and cooled (0°C) solution of 1.00 part of compound 11, 1.33 parts of 1-hydroxy-1H-benzotriazole and 23.5 parts of N,N-dimethylformamide were added 0.29 parts of glacial acetic acid under an argon stream. After stirring for 5 minutes at 0°C, 1.02 parts of N,N-methanetetrabis[cyclohexanamine] were added. Stirring was continued for 1.5 hours at 0°C and for 2 days at room temperature. The reaction mixture was cooled at 0°C for 1 hour and then filtered. The filtrate was concentrated by evaporation and the residue was taken up in a saturated sodium hydrogen carbonate solution. The product was extracted with dichloromethane and the extract was washed successively with an aqueous citric acid solution 2N and a saturated sodium hydrogen carbonate solution, dried, filtered and evaporated. The residue was purified by column chromatograhpy (silica gel : dichloromethane/methanol 97.5:2.5). The eluent of the pure fraction was evaporated and the residue was crystallized from methanol. The product was dried overnight in vacuo at 82°C, yielding 0.48 parts (39.9%) of 6-acetyl-4,5,6,7-tetrahydro-5-methylimidazo[4,5,1-jk][1,4]benzodiazepin-2(1H)-one; mp. 249.3°C (compound 35).

Example 10

A mixture of 4.2 parts of compound 2, 10 parts of acetic anhydride and 10 parts of glacial acetic acid was stirred and refluxed for 4 hours. The solvent was evaporated. To the residue was added an ammomium hydroxide solution, while cooling in an ice-bath, and the product was extracted three times with 75 parts of trichloromethane. The combined extracts were dried, filtered and evaporated. The solid residue was shaken in 40 parts of methanol, filtered off again and recrystallized three times from 20 parts of 2-methoxyethanol, yielding 2.5 parts of 1-acetyl-6-(phenylmethyl)-4,5,6,7-tetrahydroimidazo[4,5,1-jk][1,4]benzodiazepin-2(1H)-one; mp. 168-173°C (compound 3).

Example 11

A mixture of 5.6 parts of compound 2, 1.05 parts of a sodium hydride dispersion 50% in 64 parts of methylbenzene was stirred until no more hydrogen was evolved. Then it was stirred at reflux for 30 minutes. The methylbenzene layer was evaporated. To the residue was added a solution of 3.4 parts of methyliodide in 40 parts of N,N-dimethylformamide. The whole was stirred for one hour without heating and then for 15 minutes at 50°C. N,N-dimethylformamide was evaporated. To the residue were added 50 parts of water and the product was extracted twice with 75 parts of trichloromethane. The combined extracts were dried, filtered and evaporated. The residue was dissolved in 30 parts of a hydrochloric acid solution and 30 parts of water. This solution was washed with 50 parts of ligroine and treated with activated charcoal. The solution was alkalized with ammonium hydroxide and the product was extracted twice with 40 parts of methylbenzene. The combined extracts were dried, filtered and evaporated. From the oily free base, the oxalate salt was prepared in the usual manner. The crude salt was filtered off, recrystallized from methanol and dried, yielding 2.7 parts of 6-benzyl-4,5,6,7-tetrahydro-1-methylimidazo-[4,5,1-jk][1,4]-benzodiazepin-2(1H)-one oxalate; mp. 200-202.5°C (compound 4).

Example 12

A mixture of 4 parts of compound 9, 10 parts of a formaldehyde solution 40% in water and 40 parts of 2-propanol was stirred and refluxed for 16 hours. The solvent was evaporated. To the residue were added 20 parts of 4-methyl-2-pentanone and the latter was evaporated again. The residue was then dissolved in 40 parts of 2-propanone and an excess of 2-propanol previously saturated with gaseous hydrogen chloride was added to the solution. The precipitated product was filtered off, dried and recrystallized from 25 parts of water, yielding 3.7 parts of 4,5,6,7-tetrahydro-1-(hydroxymethyl)-6-(2-phenylethyl)imidazo-[4,5,1-jk][1,4]-benzodiazepin-2(1H)-one hydrochloride; mp. 217.5°C (compound 10).

All other compounds listed in the following table were prepared following the procedure of the example referred to in the column Ex. No.

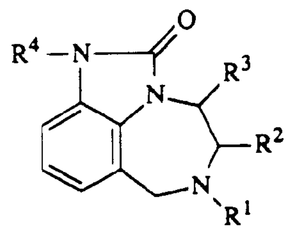

| Comp. No. | Ex. No | $R^1$ | $R^2$ | $R^3$ | $R^4$ | physical data |
|---|---|---|---|---|---|---|
| 1 | 5 | H | H | H | H | mp. 208-220°C |
| 2 | 4 | $CH_2C_6H_5$ | H | H | H | HCl/mp. 258.5-262°C (dec.) |
| 3 | 10 | $CH_2C_6H_5$ | H | H | $COCH_3$ | mp. 168-173°C |
| 4 | 11 | $CH_2C_6H_5$ | H | H | $CH_3$ | $(COOH)_2$/mp. 200-202.5°C |
| 5 | 7 | $CH_2$-CH=$CH_2$ | H | H | H | HCl/mp. 244.5°C |
| 6 | 12 | $CH_2$-CH=$CH_2$ | H | H | $CH_2OH$ | HCl/mp. 174°C |
| 7 | 7 | $C_7H_{15}$-n | H | H | H | $(COOH)_2$/mp. 220°C |
| 8 | 12 | $C_7H_{15}$-n | H | H | $CH_2OH$ | $(COOH)_2$/mp. 217°C |
| 9 | 7 | $(CH_2)_2C_6H_5$ | H | H | H | mp. 165.5°C |
| 10 | 12 | $(CH_2)_2C_6H_5$ | H | H | $CH_2OH$ | HCl/mp. 217.5°C |
| 11 | 5 | H | $CH_3$ | H | H | mp. 190.5°C |
| 12 | 6 | H | $CH_3$ | H | H | (+)-(S)/mp. 206.5°C |
| 13 | 6 | H | $CH_3$ | H | H | (-)-(R)/mp. 207.8°C |
| 14 | 4 | $CH_2C_6H_5$ | $CH_3$ | H | H | mp. 205°C |
| 15 | 7 | $(CH_2)_2C_6H_5$ | $CH_3$ | H | H | mp. 150°C |
| 16 | 8 | $C_2H_5$ | $CH_3$ | H | H | mp. 143.2°C |
| 17 | 8 | $C_3H_7$-n | $CH_3$ | H | H | mp. 149.7°C |
| 18 | 8 | $C_3H_7$-i | $CH_3$ | H | H | mp. 165.6°C |
| 19 | 8 | $C_4H_9$-n | $CH_3$ | H | H | mp. 138.0°C |
| 20 | 8 | $C_4H_9$-i | $CH_3$ | H | H | 0.5 $H_2O$/mp. 119.7°C |
| 21 | 7 | $C_7H_{15}$-n | $CH_3$ | H | H | $(COOH)_2$/mp. 163.5°C |
| 22 | 7 | $CH_2$-CH=$CH_2$ | $CH_3$ | H | H | mp. 138°C |
| 23 | 8 | $CH_2$-CH=$CH_2$ | $CH_3$ | H | H | (+)-(S)/mp. 113.8°C |
| 24 | 8 | $CH_2$-CH=$CH_2$ | $CH_3$ | H | H | (-)-(R)/mp. 113.2°C |
| 25 | 8 | $CH_2$-C($CH_3$)=$CH_2$ | $CH_3$ | H | H | mp. 150.1°C |

| Comp. No. | Ex. No. | $R^1$ | $R^2$ | $R^3$ | $R^4$ | physical data |
|---|---|---|---|---|---|---|
| 26 | 8 | $CH_2$-C($CH_3$)=$CH_2$ | $CH_3$ | H | H | (+)-(S)/mp. 152.4°C |
| 27 | 8 | ($CH_2$)$_2$-CH=$CH_2$ | $CH_3$ | H | H | mp. 107.2°C |
| 28 | 8 | $CH_2$-CH=C($CH_3$)$_2$ | $CH_3$ | H | H | (±)/mp. 158.0°C |
| 29 | 8 | $CH_2$-CH=CH-$CH_3$ | $CH_3$ | H | H | (E)/mp. 127.6°C |
| 30 | 8 | $CH_2$-CH=CH-$CH_3$ | $CH_3$ | H | H | (Z)/mp. 106.0°C |
| 31 | 8 | $CH_2$-C($CH_3$)=$CHCH_3$ | $CH_3$ | H | H | (E)/mp. 151.6°C |
| 32 | 8 | $CH_2$-C≡CH | $CH_3$ | H | H | mp. 146.0°C |
| 33 | 8 | $CH_2$-$C_3H_5$-c | $CH_3$ | H | H | $H_2O$/mp. 97.6°C |
| 34 | 8 | $CH_2$-C≡N | $CH_3$ | H | H | mp. 193.1°C |
| 35 | 9 | $COCH_3$ | $CH_3$ | H | H | mp. 249.3°C |
| 36 | 8 | $CH_2$-$CH_2$-OH | $CH_3$ | H | H | mp. 156.2°C |
| 37 | 6 | H | $C_2H_5$ | H | H | mp. 148.5°C |
| 38 | 8 | $CH_2$-C($CH_3$)=$CH_2$ | $C_2H_5$ | H | H | (±)/mp. 120.5°C |
| 39 | 6 | H | $C_3H_7$-n | H | H | mp. 156°C |
| 40 | 8 | $CH_2$-C($CH_3$)=$CH_2$ | $C_3H_7$-n | H | H | mp. 116°C |
| 41 | 6 | H | $C_3H_7$-i | H | H | mp. 162.3°C |
| 42 | 8 | $CH_2$-C($CH_3$)=$CH_2$ | $C_3H_7$-i | H | H | (±)/mp. 146.7°C |
| 43 | 6 | H | H | $CH_3$ | H | mp. 220.2°C |
| 44 | 8 | $CH_2$-C($CH_3$)=$CH_2$ | H | $CH_3$ | H | mp. 142.1°C |

C) Composition Examples

The following formulations exemplify typical pharmaceutical compositions in dosage unit form suitable for systemic administration to warm-blooded animals in accordance with the present invention.

"Active ingredient" (A.I.) as used throughout these examples relates to a compound of formula (I), a pharmaceutically acceptable acid addition salt or a stereochemically isomeric form thereof.

Example 13 : ORAL DROPS

500 g of the A.I. was dissolved in 0.5 l of 2-hydroxypropanoic acid and 1.5 l of the polyethylene glycol at 60~80°C. After cooling to 30~40°C there were added 35 l of polyethylene glycol and the mixture was stirred well. Then there was added a solution of 1750 g of sodium saccharin in 2.5 l of purified water and while stirring there were added 2.5 l of cocoa flavor and polyethylene glycol q.s. to a volume of 50 l, providing an oral drop solution comprising 10 mg/ml of the A.I. The resulting solution was filled into suitable containers.

Example 14 : ORAL SOLUTION

9 g of methyl 4-hydroxybenzoate and 1 part of propyl 4-hydroxybenzoate were dissolved in 4 l of boiling purified water. In 3 l of this solution were dissolved first 10 g of 2,3-dihydroxybutanedioic acid and thereafter 20 g of the A.I. The latter solution was combined with the remaining part of the former solution and 12 l 1,2,3-propanetriol and 3 l of sorbitol 70% solution were added thereto. 40 g of sodium saccharin were dissolved in 0.5 l of water and 2 ml of raspberry and 2 ml of gooseberry essence were added. The latter solution was combined with the former, water was added q.s. to a volume of 20 l providing an oral solution comprising 5 mg of

the A.I. per teaspoonful (5 ml). The resulting solution was filled in suitable containers.

Example 15 : CAPSULES

20 g of the A.I., 6 g sodium lauryl sulfate, 56 g starch, 56 g lactose, 0.8 g colloidal silicon dioxide, and 1.2 g magnesium stearate were vigorously stirred together. The resulting mixture was subsequently filled into 1000 suitable hardened gelatin capsules, each comprising 20 mg of the A.I..

Example 16 : FILM-COATED TABLETS

Preparation of tablet core

A mixture of 100 g of the A.I., 570 g lactose and 200 g starch was mixed well and thereafter humidified with a solution of 5 g sodium dodecyl sulfate and 10 g polyvinylpyrrolidone (Kollidon-K 90®) in about 200 ml of water. The wet powder mixture was sieved, dried and sieved again. Then there was added 100 g microcrystalline cellulose (Avicel®) and 15 g hydrogenated vegetable oil (Sterotex®). The whole was mixed well and compressed into tablets, giving 10.000 tablets, each comprising 10 mg of the active ingredient.

Coating

To a solution of 10 g methyl cellulose (Methocel 60 HG®) in 75 ml of denaturated ethanol there was added a solution of 5 g of ethyl cellulose (Ethocel 22 cps®) in 150 ml of dichloromethane. Then there were added 75 ml of dichloromethane and 2.5 ml 1,2,3-propanetriol. 10 g of polyethylene glycol was molten and dissolved in 75 ml of dichloromethane. The latter solution was added to the former and then there were added 2.5 g of magnesium octadecanoate, 5 g of polyvinylpyrrolidone and 30 ml of concentrated colour suspension (Opaspray K-1-2109®) and the whole was homogenated. The tablet cores were coated with the thus obtained mixture in a coating apparatus.

Example 17 : INJECTABLE SOLUTION

1.8 g methyl 4-hydroxybenzoate and 0.2 g propyl 4-hydroxybenzoate were dissolved in about 0.5 l of boiling water for injection. After cooling to about 50°C there were added while stirring 4 g lactic acid, 0.05 g propylene glycol and 4 g of the A.I.. The solution was cooled to room temperature and supplemented with water for injection q.s. ad 1 l volume, giving a solution of 4 mg A.I. per ml. The solution was sterilized by filtration (U.S.P. XVII p. 811) and filled in sterile containers.

Example 18 : SUPPOSITORIES

3 g A.I. was dissolved in a solution of 3 g 2,3-dihydroxybutanedioic acid in 25 ml polyethylene glycol 400. 12 g surfactant (SPAN®) and triglycerides (Witepsol 555®) q.s. ad 300 g were molten together. The latter mixture was mixed well with the former solution. The thus obtained mixture was poured into moulds at a temperature of 37-38°C to form 100 suppositories each containing 30 mg of the A.I.

## Claims

1. A chemical compound having the formula

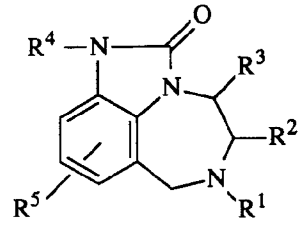

(I)

a pharmaceutically acceptable acid addition salt or a stereochemically isomeric forms thereof, wherein

$R^1$ is hydrogen, $C_{1-8}$alkyl, $C_{3-6}$alkenyl, $C_{3-6}$alkynyl, $C_{1-6}$alkylcarbonyl, $C_{3-6}$cycloalkyl, or $C_{1-6}$alkyl substituted with aryl, hydroxy, cyano or $C_{3-6}$cycloalkyl;

$R^2$ is hydrogen, $C_{1-6}$alkyl or $C_{3-6}$alkenyl;

$R^3$ is hydrogen or $C_{1-6}$alkyl;

$R^4$ is hydrogen; $C_{1-6}$alkyl optionally substituted with hydroxy, cyano, hydroxycarbonyl or $C_{1-6}$alkoxycarbonyl; $C_{1-6}$alkylcarbonyl; $C_{3-6}$alkenyl; $C_{3-6}$cycloalkyl; $C_{5-6}$cycloalkenyl;

$R^5$ is hydrogen, $C_{1-6}$alkyl or halo; and

aryl is phenyl optionally substituted with up to 3 substituents independently selected from $C_{1-6}$alkyl, halo, hydroxy, $C_{1-6}$alkyloxy, amino, nitro and trifluoromethyl.

2. A chemical compound according to claim 1 wherein
$R^1$ is hydrogen, $C_{1-8}$alkyl, $C_{3-6}$alkenyl, $C_{3-6}$alkynyl, $C_{1-6}$alkylcarbonyl or $C_{1-6}$alkyl substituted with aryl, hydroxy, cyano or $C_{3-6}$cycloalkyl;
$R^2$ is hydrogen or $C_{1-6}$alkyl; and
$R^4$ is hydrogen; $C_{1-6}$alkyl optionally substituted with hydroxy; or $C_{1-6}$alkylcarbonyl.

3. A chemical compound according to claim 2 wherein
$R^1$ is $C_{1-8}$alkyl, $C_{3-6}$alkenyl, $C_{3-6}$alkynyl, or $C_{1-6}$alkyl substituted with aryl, hydroxy, cyano or $C_{3-6}$cycloalkyl;
$R^4$ is hydrogen; and
$R^5$ is hydrogen.

4. A chemical compound according to claim 1, wherein the compound of formula (I) is selected from 4,5,6,7-tetrahydro-5-methyl-6-(2-propenyl)imidazo-[4,5,1-jk][1,4]benzodiazepin-2(1H)-one; 4,5,6,7-tetrahydro-5-methyl-6-(2-methyl-2-propenyl)imidazo[4,5,1-jk][1,4]benzodiazepin-2(1H)-one; (+)-(S)-4,5,6,7-tetrahydro-5-methyl-6-(2-propenyl)imidazo[4,5,1-jk][1,4]benzodiazepin-2(1H)-one; 6-(3-butenyl)-4,5,6,7-tetrahydro-5-methylimidazo[4,5,1-jk][1,4]benzodiazepin-2(1H)-one; 4,5,6,7-tetrahydro-5-methyl-6-propylimidazo[4,5,1-jk][1,4]benzodiazepin-2(1H)-one; (+)-(S)-4,5,6,7-tetrahydro-5-methyl-6-(2-methyl-2-propenyl)imidazo[4,5,1-jk][1,4]-benzodiazepin-2(1H)-one; 6-(cyclopropylmethyl)-4,5,6,7-tetrahydro-5-methylimidazo-[4,5,1-jk][1,4]benzodiazepin-2(1H)-one monohydrate and 4,5,6,7-tetrahydro-5-methyl-6-(3-methyl-2-butenyl)imidazo [4,5,1-jk][1,4]benzodiazepin-2(1H)-one.

5. An antiviral composition comprising a pharmaceutically acceptable carrier and as active ingredient an effective antiviral amount of a compound of formula (I) as claimed in any of claims 1 to 4.

6. A process for preparing a composition as claimed in claim 6, characterized in that the active ingredient is intimately mixed with the carrier.

7. A compound of formula (I) as claimed in any of claims 1 to 4 for use as a medicine.

8. A compound of formula (I) as claimed in any of claims 1 to 4 for use as an anti-viral medicine.

9. A process of preparing a chemical compound of formula (I) as claimed in any of claims 1 to 4, characterized by
a)condensing a 9-amino-2,3,4,5-tetrahydro-1H-1,4-benzodiazepine of formula

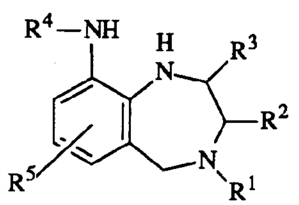

(II)

wherein $R^1$, $R^2$, $R^3$, $R^4$ and $R^5$ are as defined in formula (I),
with a carbonyl-generating reagent of formula L-C(=O)-L (III), wherein L is a leaving group, optionally in a reaction-inert solvent and in the presence of a base;
b) hydrogenolyzing a benzylated compound of formula

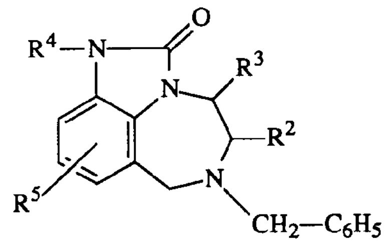

(I-b)

wherein $R^2$, $R^3$, $R^4$ and $R^5$ are as defined in formula (I), in a reaction-inert solvent in the presence of a metal catalyst and under a hydrogen atmosphere, thus yielding a compound of formula

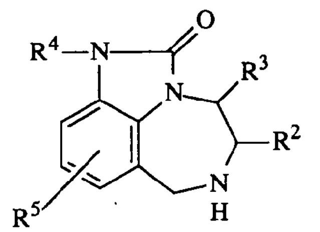

(I-a)

wherein $R^1$ is hydrogen and $R^2$, $R^3$, $R^4$ and $R^5$ are as defined in formula (I); and in order to prepare compounds of formula (I) wherein $R^1$ is other than hydrogen, N-alkylating said compound of formula (I-a) with a reagent of formula $R^{1-a}$-W (IV), wherein W represents a reactive leaving group and $R^{1-a}$ is $R^1$ as defined in formula (I), but other than hydrogen, in a reaction-inert solvent in the presence of a base and optionally of an iodide salt, thus yielding a compound of formula

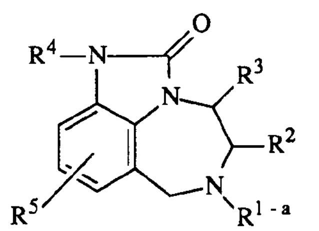

(I-c)

wherein $R^2$, $R^3$, $R^4$ and $R^5$ are as defined in formula (I) and $R^{1-a}$ is as defined hereinabove; or alternatively, N-alkylating said compound of formula (I-a) reductively with a ketone or aldehyde of formula $R^{1-b}$=O (V), wherein $R^{1-b}$ represents a geminal bivalent radical derived from $R^{1-a-1}$-H wherein two geminal hydrogen atoms are replaced by =O and $R^{1-a-1}$ is as $R^1$, but other than hydrogen, $C_{3-6}$alkenyl or $C_{3-6}$alkynyl, in a reaction-inert organic solvent, with a reducing agent or under a hydrogen atmosphere in the presence of a catalyst, thus yielding a compound of formula

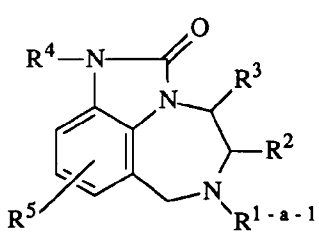

(I-d)

wherein $R^2$, $R^3$, $R^4$ and $R^5$ are as defined in formula (I) and $R^{1-a-1}$ is as defined hereinabove;
d) N-alkylating or N-acylating a compound of formula

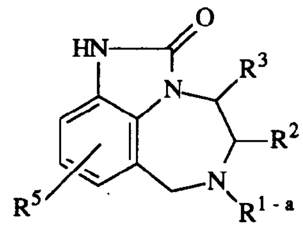

(I-f)

wherein $R^2$, $R^3$ and $R^5$ are as defined in formula (I), $R^{1-a}$ is as defined hereinabove and $R^4$ is hydrogen, with a reagent of formula $R^{4-a}$-W (VI) wherein W is a reactive leaving group and $R^{4-a}$ is $R^4$ as defined under formula (I), but other than hydrogen, thus yielding a compound of formula

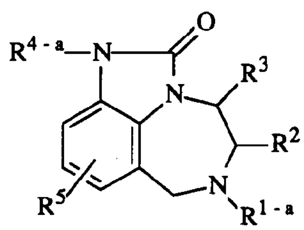

(I-e)

wherein $R^2$, $R^3$ and $R^5$ are as defined in formula (I) and $R^{1-a}$ and $R^{4-a}$ are as defined hereinabove; and

if desired, converting the compounds of formula (I) into each other following art-known procedures of functional group transformation; and,

if further desired, converting a compound of formula (I) into a therapeutically active non-toxic acid addition salt form by treatment with an acid; or conversely, converting the acid salt into the free base form with alkali; and/or preparing stereochemically isomeric forms thereof.

## Patentansprüche

1. Chemische Verbindung mit der Formel

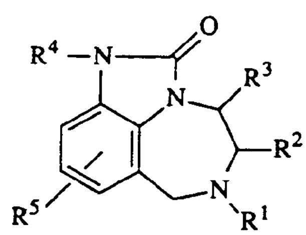

(I) ,

ein pharmazeutisch annehmbares Säureadditionssalz oder eine stereochemisch isomere Form hievon, worin

$R^1$ für Wasserstoff, $C_{1-8}$-Alkyl, $C_{3-6}$-Alkenyl, $C_{3-6}$-Alkinyl, $C_{1-6}$-Alkylcarbonyl, $C_{3-6}$-Cycloalkyl oder $C_{1-6}$-Alkyl , das durch Aryl, Hydroxy, Cyano oder $C_{3-6}$-Cycloalkyl substituiert ist, steht;

$R^2$ Wasserstoff, $C_{1-6}$-Alkyl oder $C_{3-6}$-Alkenyl bedeutet;

$R^3$ für Wasserstoff oder $C_{1-6}$-Alkyl steht;

$R^4$ Wasserstoff, $C_{1-6}$-Alkyl, das gegebenenfalls durch Hydroxy, Cyano, Hydroxycarbonyl oder $C_{1-6}$-Alkoxycarbonyl substituiert ist; $C_{1-6}$-Alkylcarbonyl; $C_{3-6}$-Alkenyl; $C_{3-6}$-Cycloalkyl oder $C_{5-6}$-Cycloalkenyl darstellt;

$R^5$ für Wasserstoff, $C_{1-6}$-Alkyl oder Halogen steht; und

Aryl für Phenyl steht, das gegebenenfalls mit bis zu 3 Substituenten, unabhängig voneinander ausgewählt unter $C_{1-6}$-Alkyl, Halogen, Hydroxy, $C_{1-6}$-Alkyloxy, Amino, Nitro und Trifluormethyl, substituiert ist.

2. Chemische Verbindung nach Anspruch 1, worin

$R^1$ für Wasserstoff, $C_{1-8}$-Alkyl, $C_{3-6}$-Alkenyl, $C_{3-6}$-Alkinyl, $C_{1-6}$-Alkylcarbonyl oder $C_{1-6}$-Alkyl , das mit Aryl, Hydroxy, Cyano oder $C_{3-6}$-Cycloalkyl subsituiert ist, steht;

$R^2$ Wasserstoff oder $C_{1-6}$-Alkyl bedeutet; und

$R^4$ für Wasserstoff; gegebenenfalls durch Hydroxy substituiertes $C_{1-6}$-Alkyl; oder $C_{1-6}$-Alkylcarbonyl steht.

**3.** Chemische Verbindung nach Anspruch 2, worin

$R^1$ für $C_{1-8}$-Alkyl, $C_{3-6}$-Alkenyl, $C_{3-6}$-Alkinyl oder durch Aryl, Hydroxy, Cyano oder $C_{3-6}$-Cycloalkyl substituiertes $C_{1-6}$-Alkyl steht;

$R^4$ Wasserstoff bedeutet; und

$R^5$ Wasserstoff ist.

**4.** Chemische Verbindung nach Anspruch 1, worin die Verbindung der Formel (I) unter 4,5,6,7-Tetrahydro-5-methyl-6-(2-propenyl)imidazo[4,5,1-jk]-[1,4]benzodiazepin-2(1H)-on; 4,5,6,7-Tetrahydro-5-methyl-6-(2-methyl-2-propenyl)imidazo[4,5,1-jk][1,4]benzodiazepin-2(1H)-on; (+)-(S)-4,5,6,7-Tetrahydro-5-methyl-6-(2-propenyl)imidazo[4,5,1-jk][1,4]benzodiazepin-2 (1H)-on; 6-(3-Butenyl)-4,5,6,7-tetrahydro-5-methylimidazo[4,5,1-jk]-[1,4]benzodiazepin-2(1H)-on; 4,5,6,7-Tetrahydro-5-methyl-6-propylimidazo[4,5,1-jk][1,4]benzodiazepin-2(1H)-on; (+)-(S)-4,5,6,7-Tetrahydro-5-methyl-6-(2-methyl-2-propenyl)imidazo[4,5,1-jk][1,4]benzodiazepin-2(1H)-on; 6-(Cyclopropylmethyl)-4,5,6,7-tetrahydro-5-methylimidazol[4,5,1-jk][1,4]benzodiazepin-2(1H)-on-monhydrat und 4,5,6,7-Tetrahydro-5-methyl-6-(3-methyl-2-butenyl)imidazo[4,5,1-jk][1,4]benzodiazepin-2(1H)-on ausgewählt ist.

**5.** Antivirale Zusammensetzung, umfassend einen pharmazeutisch annehmbaren Träger und als wirksamen Bestandteil eine wirksame antivirale Menge einer Verbindung der Formel (I), wie in einem der Ansprüche 1 bis 4 beansprucht.

**6.** Verfahren zur Herstellung einer Zusammensetzung nach Anspruch 5, dadurch gekennzeichnet, daß der wirksame Bestandteil innig mit dem Träger vermischt wird.

**7.** Eine Verbindung der Formel (I) nach einem der Ansprüche 1 bis 4 zur Anwendung als ein Medikament.

**8.** Eine Verbindung der Formel (I) nach einem der Ansprüche 1 bis 4 zur Anwendung als ein antivirales Medikament.

**9.** Verfahren zur Herstellung einer chemischen Verbindung der Formel (I) nach einem der Ansprüche 1 bis 4, gekennzeichnet durch

a) Kondensieren eines 9-Amino-2,3,4,5-tetrahydro-1H-1,4-benzodiazepins der Formel

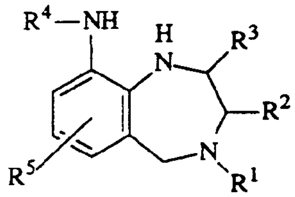

(II) ,

worin $R^1$, $R^2$, $R^3$, $R^4$ und $R^5$ wie in Formel (I) definiert sind, mit einem Carbonyl-bildenden Reagens der Formel L-C(=O)-L (III), worin L eine Leaving-Gruppe ist, gegebenenfalls in einem reaktionsinerten Lösungsmittel und in Anwesenheit einer Base;

b) Hydrogenolysierern einer benzylierten Verbindung der Formel

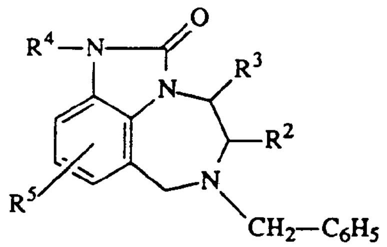 (I-b) ,

worin $R^2$, $R^3$, $R^4$ und $R^5$ wie in Formel (I) definiert sind, in einem reaktionsinerten Lösungsmittel in Anwesenheit eines Metallkatalysators und unter einer Wasserstoffatmosphäre, unter Ausbildung einer Verbindung der Formel

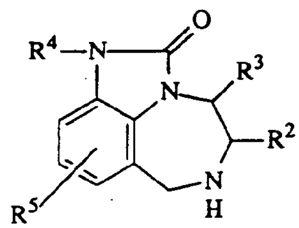 (I-a) ,

worin $R^1$ Wasserstoff bedeutet und $R^2$, $R^3$, $R^4$ und $R^5$ wie in Formel (I) definiert sind; und, zur Herstellung von Verbindungen der Formel (I), worin $R^1$ eine von Wasserstoff verschiedene Bedeutung hat, N-Alkylieren dieser Verbindung der Formel (I-a) mit einem Reagens der Formel $R^{1-a}$-W (IV), worin W eine reaktionsfähige Leaving-Gruppe bedeutet und $R^{1-a}$ wie in Formel (I) für $R^1$ definiert ist, aber eine andere Bedeutung als Wasserstoff hat, in einem reaktionsinerten Lösungsmittel in Anwesenheit einer Base und gegebenenfalls eines Iodidsalzes, unter Ausbildung einer Verbindung der Formel

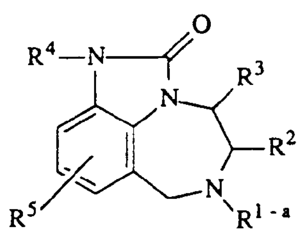 (I-c) ,

worin $R^2$, $R^3$, $R^4$ und $R^5$ wie in Formel (I) definiert sind und $R^{1-a}$ wie vorstehend definiert ist; oder in alternativer Weise reduktives N-Alkylieren dieser Verbindung der Formel (I-a) mit einem Keton oder Aldehyd der Formel $R^{1-b}$=O (V), worin $R^{1-b}$ einen geminalen zweiwertigen Rest bedeutet, der von $R^{1-a-1}$-H abgeleitet ist, worin zwei geminale Wasserstoffatome durch =O ersetzt sind und $R^{1-a-1}$ die gleiche Bedeutung wie $R^1$ hat, aber von Wasserstoff, $C_{3-6}$-Alkenyl oder $C_{3-6}$-Alkinyl verschieden ist, in einem reaktionsinerten organischen Lösungsmittel mit einem Reduktionsmittel oder unter einer Wasserstoffatmosphäre in Anwesenheit eines Katalysators, unter Ausbildung einer Verbindung der Formel

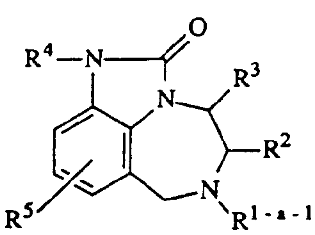 (I-d) ,

worin $R^2$, $R^3$, $R^4$ und $R^5$ wie in Formel (I) definiert sind und $R^{1-a-1}$ wie vorstehend definiert ist;
c) N-Alkylieren oder N-Acylieren einer Verbindung der Formel

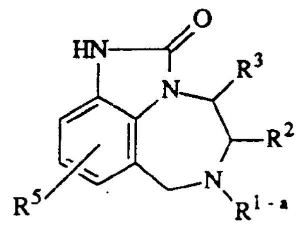

(I-f) ,

worin $R^2$, $R^3$ und $R^5$ wie in Formel (I) definiert sind, $R^{1-a}$ wie vorstehend definiert ist und $R^4$ Wasserstoff bedeutet, mit einem Reagens der Formel $R^{4-a}$-W (VI), worin W eine reaktionsfähige Leaving-Gruppe bedeutet und $R^{4-a}$ wie $R^4$ unter Formel (I) definiert ist, aber eine andere Bedeutung als Wasserstoff hat, unter Ausbildung einer Verbindung der Formel

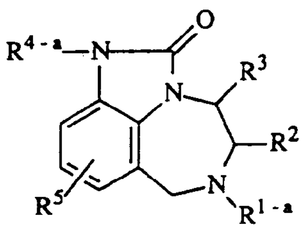

(I-e) ,

worin $R^2$, $R^3$ und $R^5$ wie in Formel (I) definiert sind und $R^{1-a}$ und $R^{4-a}$ wie zuvor definiert sind; und

falls gewünscht, Überführen der Verbindungen der Formel (I) ineinander nach bekannten Methoden der Umwandlung funktioneller Gruppen; und,

falls weiter gewünscht, Überführen einer Verbindung der Formel (I) in eine therapeutisch wirksame nicht-toxische Säureadditionssalzform durch Behandlung mit einer Säure; oder umgekehrt, Überführen des Säuresalzes in die freie Basenform mit Alkali; und/oder Herstellen stereochemisch isomerer Formen hievon.

**Revendications**

1. Composé chimique ayant la formule

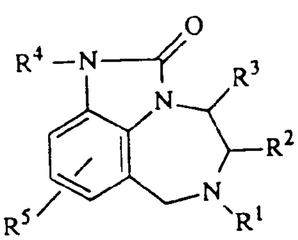

(I)

l'un des ses sels d'addition avec un acide pharmaceutiquement acceptables ou l'un de ses formes isomères d'un point de vue stéréochimique, où :

$R^1$ est un hydrogène ou un radical alkyle en $C_{1-8}$, alcényle en $C_{3-6}$, alcynyle en $C_{3-6}$, (alkyle en $C_{1-6}$)-carbonyle, cycloalkyle en $C_{3-6}$ ou alkyle en $C_{1-6}$ substitué par des radicaux aryle, hydroxy, cyano ou cycloalkyle en $C_{3-6}$ ;

$R^2$ est un hydrogène ou un radical alkyle en $C_{1-6}$ ou alcényle en $C_{3-6}$ ;

$R^3$ est un hydrogène ou un radical alkyle en $C_{1-6}$ ;

$R^4$ est un hydrogène ; un radical alkyle en $C_{1-6}$ éventuellement substitué par des radicaux hydroxy, cyano, hydroxycarbonyle ou (alcoxy en $C_{1-6}$)-carbonyle ; (alkyle en $C_{1-6}$)-carbonyle ; alcényle en $C_{3-6}$ ; cycloalkyle en $C_{3-6}$ ; cycloalcényle en $C_{5-6}$ ;

$R^5$ est un hydrogène ou un radical alkyle en $C_{1-6}$ ou halogéno ; et

aryl représente un radical phényle éventuellement substitué ayant jusqu'à 3 substituants choisis,

indépendamment les uns des autres, chacun parmi les radicaux alkyle en $C_{1-6}$, halogéno, hydroxy, alkyloxy en $C_{1-6}$, amino, nitro et trifluorométhyle.

2. Composé chimique selon la revendication 1, dans lequel
   $R^1$ est un hydrogène ou un radical alkyle $C_{1-8}$, alcényle en $C_{3-6}$, alcynyle en $C_{3-6}$, (alkyle en $C_{1-6}$)-carbonyle ou alkyle en $C_{1-6}$ substitué par des radicaux aryle, hydroxy, cyano ou cycloalkyle en $C_{3-6}$ ;
   $R^2$ est un hydrogène ou un radical alkyle en $C_{1-6}$ ; et
   $R^4$ est un hydrogène ; un radical alkyle en $C_{1-6}$ éventuellement substitué par des radicaux hydroxy ; ou encore (alkyle en $C_{1-6}$)-carbonyle.

3. Composé chimique selon la revendication 2, dans lequel
   $R^1$ est un radical alkyle en $C_{1-8}$, alcényle en $C_{3-6}$, alcynyle en $C_{3-6}$, ou encore alkyle en $C_{1-6}$ substitué par des radicaux aryle, hydroxy, cyano ou cycloalkyle en $C_{3-6}$ ;
   $R^4$ est un hydrogène ; et
   $R^5$ est un hydrogène.

4. Composé chimique selon la revendication 1, où le composé de formule (I) est choisi parmi les composés suivants : 4,5,6,7-tétrahydro-5-méthyl-6-(2-propényl)imidazo [4,5,1-jk][1,4]benzodiazépine-2-(1H)-one;4,5,6,7-tétrahydro-5-méthyl-6-(2-méthyl-2-propényl)imidazo[4,5,1-jk][1,4]-benzodiazépine-2(1H)-one; (+)-(S)-4,5,6,7-tétrahydro-5-méthyl-6-(2-propényl)imidazo[4,5,1-jk][1,4]benzodiazépine-2-1H)-one; 6-(3-butény)-4,5,6,7-tétrahydro-5-méthyl-imidazo[4,5,1-jk][1,4]benzodiazépine-2(1H)-one; 4,5,6,7-tétrahydro-5-méthyl-6-propylimidazo[4,5,1-jk][1,4]-benzodiazépine-2(1H)-one;(+)-(S)-4,5,6,7-tétrahydro-5-méthyl-6-(2-méthyl-2-propényl)imidazo[4,5,1-jk][1,4]benzodiazépine-2(1H)-one; 6-(cyclopropylméthyl)-4,5,6,7-tétrahydro-5-méthylimidazo[4,5,1-jk][1,4]benzodiazépine-2(1H)-one monohydrate ; et 4,5,6,7-tétrahydro-5-méthyl-6-(3-méthyl-2-butényl)imidazo[4,5,1-jk][1,4]benzodiazépine-2(1H)-one.

5. Composition antivirale comprenant un excipient pharmaceutiquement acceptable et, en tant que principe actif, une quantité à effet antiviral d'un composé de formule (I) selon l'une quelconque des revendications 1 à 4.

6. Procédé de préparation d'une composition selon la revendication 6, caractérisé en ce que le principe actif est intimement mélangé à l'excipient.

7. Composé de formule (I) selon l'une quelconque des revendications 1 à 4, pour utilisation comme médicament.

8. Composé de formule (I) selon l'une quelconque des revendications 1 à 4, pour utilisation comme médicament antiviral.

9. Procédé de préparation d'un composé chimique de formule (I) selon l'une quelconque des revendications 1 à 4, caractérisé en ce qu'il comprend les étapes consistant :
   a) à condenser une 9-amino-2,3,4,5-tétrahydro-1H-1,4-benzodiazépine de formule

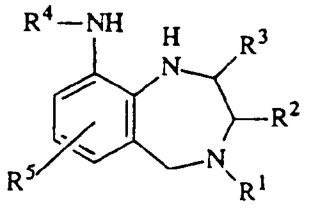

(II)

dans laquelle $R^1$, $R^2$, $R^3$, $R^4$ et $R^5$ sont tels que définis pour la formule (I),
avec un réactif produisant des radicaux carbonyle, de formule L-C(=O)-L (III), où L est un groupe éliminable, éventuellement dans un solvant inerte vis-à-vis de la réaction et en présence d'une base ;
   b) à hydrogénolyser un composé benzylé de formule

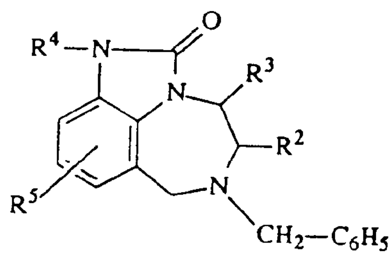

(I-b)

dans laquelle $R^2$, $R^3$, $R^4$ et $R^5$ sont tels que définis pour la formule (I), dans un solvant inerte vis-à-vis de la réaction en présence d'un catalyseur métallique et sous atmosphére d'hydrogène, pour obtenir un composé de formule

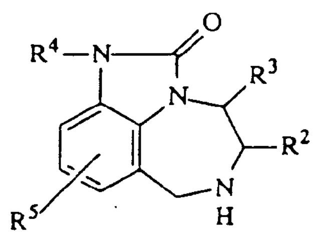

(I-a)

dans laquelle $R^1$ est un hydrogène et $R^2$, $R^3$, $R^4$ et $R^5$ sont tels que définis pour la formule (I) ; et, pour préparer des composés de formule (I) dans laquelle $R^1$ est autre qu'un hydrogène, à N-alkyler ledit composé de formule (I-a) avec un réactif de formule $R^{1-a}$-W (IV) où W représente un groupe éliminable réactif et $R^{1-a}$ est un radical $R^1$ tel que défini pour la formule (I) mais autre qu'un hydrogène, dans un solvant inerte vis-à-vis de la réaction, en présence d'une base et éventuellement d'un sel iodure, de façon à obtenir un composé de formule

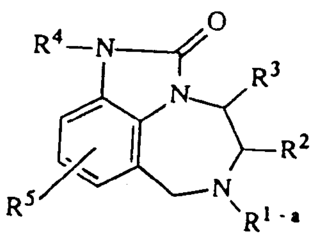

(I-c)

dans laquelle $R^2$, $R^3$, $R^4$ et $R^5$ sont tels que définis pour la formule (I) et $R^{1-a}$ est tel que défini ci-dessus ; ou encore à N-alkyler ledit composé de formule (I-a) par réduction avec une cétone ou un aldéhyde de formule $R^{1-b}$=O (V), où $R^{1-b}$ représente un radical bivalent géminé dérivant du radical $R^{1-a-1}$-H où deux atomes d'hydrogène géminé sont remplacé par =O, et $R^{1-a-1}$ est tel que le radical $R^1$ mais autre qu'un hydrogène, un radical alcényle en $C_{3-6}$ ou alcynyle en $C_{3-6}$, dans un solvant organique inerte vis-à-vis vis de la réaction, avec un réducteur ou sous une atmosphère d'hydrogène en présence d'un catalyseur, pour obtenir un composé de formule

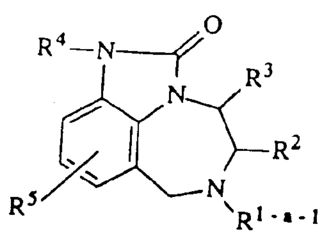

(I-d)

dans laquelle $R^2$, $R^3$, $R^4$ et $R^5$ sont tels que définis pour la formule (I) et $R^{1-a-1}$ est tel que défini ci-dessus ;

d) à N-alkyler ou N-acyler un composé de formule

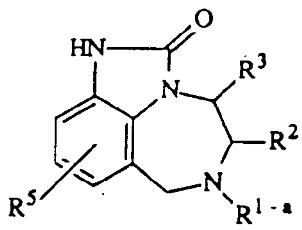

(I-f)

dans laquelle $R^2$, $R^3$ et $R^5$ sont tels que définis pour la formule (I), $R^{1-a}$ est tel que défini ci-dessus et $R^4$ est un hydrogène, avec un réactif de formule $R^{4-a}$-W (VI), où W est un groupe éliminable réactif, et $R^{4-a}$ est un radical $R^4$ tel que défini pour la formule (I) mais autre qu'un hydrogène, de façon à obtenir un composé de formule

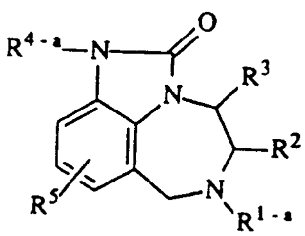

(I-e)

dans laquelle $R^2$, $R^3$ et $R^5$ sont tels que définis pour la formule (I), et $R^{1-a}$ et $R^{4-a}$ sont tels que définis ci-dessus ; et

si on le souhaite, à convertir les composés de formule (I) les uns en les autres, par des techniques connues de transformation des groupes fonctionnels ; et

si on le souhaite aussi, à convertir un composé de formule (I) en une forme non toxique thérapeutiquement active du type sel d'addition avec un acide, par traitement avec un acide ; ou inversement à convertir le sel d'addition avec un acide en la forme base libre à l'aide d'un alcali ; et/ou à en préparer des formes isomères d'un point de vue stéréochimique.